# EUROPEAN PATENT SPECIFICATION

(11) **EP 1 773 252 B1**
(45) Date of publication and mention of the grant of the patent: **18.08.2010**
(21) Application number: 05766549.9
(22) Date of filing: 11.07.2005
(51) Int. Cl.: A61F 2/42

(54) **FINGER OR TOE PROSTHESIS**
FINGER- ODER ZEHENPROTHESE
PROTHESE DE DOIGT OU D'ORTEIL

(30) Priority: 09.07.2004 BE 200400341
(43) Date of publication of application: 18.04.2007
(73) Proprietor: Otto Bock Healthcare Products GmbH, 1070 Wien (AT)
(72) Inventor: CUBBER, Jan, de, B-1930 Zaventem (BE)
(74) Representative: Stornebel, Kai
(86) International application number: PCT/EP2005/007503
(87) International publication number: WO 2006/005569

(56) References cited:
- EP-A- 1 438 937
- WO-A-01/97718
- WO-A-03/017879
- DE-C1- 19 512 854
- US-A- 4 685 924
- US-A- 5 941 914

## Description

The invention relates to a finger or toe prosthesis having an intracorporeal implant and an external modular frame constituting the core of the finger or toe prosthesis with a certain number of modules or components, each with its own function, which will be assembled to constitute one modular frame which can be covered by an aesthetical or functional cover.

The WO 03/017876 A1 discloses a drive device for a finger prosthesis of substantially natural size designed to bend the finger prosthesis about a shaft relative to a fixing, e.g. in a human or artificial metacarpus. The drive device comprises a motor which can be connected to an energy source, and a transmission intended to transform a force from the motor to the finger prosthesis to perform. the movement.

WO - 01/97718 A1 relates to a transcutaneous prosthesis comprising a first component shaped for implantation into a bone, a second component intended for location between the bone and the skin, and a third component intended for location exterior to the skin surface, having a low surface energy which deters bacterial adhesion. The third component may be connected to an artificial limb or digit.

US-A-5,941,914 relates to an articulated, stacked plate artificial finger prosthesis, which is to be articulated by a lever arm means for actuating the finger. Actuating means are provided to move the different parts of the articulated, stacked plate body part. By retracting a lever arm the articulated finger assembly is caused to bend.

EP 1 438 937 A1 relates to a finger prosthesis as defined in in the preamble of claim 1. It comprises, in particular, a stem like implant to be inserted into a tubular bone. The insert can be coupled with an extracorporeal substitute element.

It is also known that by using an osseous integration technique and silicone cosmetic prosthesis serious phalangeal amputations can be treated. This technique consists in the insertion of a threaded titanium implant in the medullar canal of the bone structure in a finger, anchoring a silicone finger prosthesis to a skin-penetrating abutment and locking the prosthesis in place by a small transverse screw device. Such an osseous integrated silicone prosthesis has good cosmetic results but cannot replace the function of the lost bone structure completely.

An object of the invention is to provide a finger or toe prosthesis which is more functional in use and which does have a more natural appearance.

The object of the invention is achieved by a finger or toe prosthesis according to claim 1 wherein the external modular frame is displaceably fixed to the intracorporeal implant. Due to the displaceable fixing it is possible to bend the external modular frame or a part of the external modular frame relative to the intracorporeal implant to adapt the form of the prosthesis to actual requirements. Furthermore the displaceable fixing allows the external modular frame to move relatively to the intracorporeal implant upon an overload so that the stress to the intracorporeal implant or the bone structure can be limited.

The external modular frame is mounted on a transcutaneous component of the implant so that a stable mechanical fixing can be achieved. Alternatively the external modular frame can be coupled to the transcutaneous component by means of a coupling component or interface. The external modular frame is positioned outside of the body and is anchored to the coupling component or interface. The coupling component can be a modular part of the modular frame fixed to it or it may be an integral part of the external modular frame. The external modular frame and the coupling component, as well as the transcutaneous component may constitute in part or in total an aesthetic finger prosthesis.

The coupling component may be an elastic component or a part of a joint means, e. g. the proximal part of a hinge joint. The coupling component may be constituted as a deformable component, e. g. a plastic device which recovers its shape after deformation.

The external modular frame has a joint or bendable interface onto which an extension may be connected. By adaption of the length of the extension, e.g. with a modular setup of the extension, the position of the joint or bendable interface of the external modular frame can be brought to its ideal position. A joint, e. g. a simple hinge, can be used for a finger prosthesis with frequent flexion or extension movements, a bendable interface is preferably used for a prosthesis, which is displaced not so often or for single adjustment.

The external modular frame is covered with a cosmetic or functional cover consisting of smooth material reversibly or irreversibly fixed to the external modular frame. The external modular frame may be covered completely by the cover wherein the cover may be composed of various materials with various physical characteristics, e.g. (visco-)elasticity and plasticity, compressibility and nonlinear deformation behaviour, for supporting the underlying modular frame and to achieve a natural appearance in respect to wrinkling, elongation, shortening or buckling. The external modular frame and the cover may be designed to become one unit. The cover may be adapted to the specific frame and its design or function and support the frame in its function. The cover may be made of foam or silicone for supporting the function of the underlying external modular frame.

Furthermore, the coupling component may be designed as an adhesive or as a snap joint.

To avoid injuries or lesions the coupling component may contain a fail safety mechanism or overload protection device so that in the event of an overload, hyper-rotation or hyper-flexion the external modular frame and the intracorporeal part will be decoupled as to safeguard the bone structure of traumata.

Should the coupling component not constitute part of the transcutaneous component or abutment, it may easily be removed with or not by means of using an accessory.

The patient may also, if desired, cover the parts proximal to the coupling component or solely transcutaneous component avoiding visibility of metal elements when the finger epithesis is being removed or protecting the transcutaneous component and adjacent tissue. This cover or protective cap may exert pressure to the skin or soft tissue or may be loaded with an agent for stimulating the skin or soft tissue. In addition the coupling component may also serve as an extension thus the level of amputation may be mitigated whereby a joint may be placed in an ideal position. Consequently, the coupling component can be adapted, e.g. by telescopic elements or production of various lengths.

The intracorporeal implant is permanently connected to the bone structure of the stump. It may be a screwed implant, which will be introduced in a longitudinal manner into the remaining bone structure. Osseous integration will ensure a definitive anchoring of the implant, preferably made of titanium, into the bone structure. The transcutaneous component, an integral part of the implant, perforates the skin and offers the possibility of connecting the other structures.

The material, of e. g. the coupling component or the cover, which gets into contact with the skin or soft tissue of the patient may be loaded with an agent released by the material and influencing the skin or soft tissue surrounding the transcutaneous component in a positive way, e.g. infection treatment.

The coupling component may exercise pressure on the tissue surrounding the transcutaneous component to stimulate the soft tissue and to provide a feedback of the exerted pressure of the prosthesis.

Preferably, the joint is lockable in its position by a form fit locking upon reaching a predetermined angle position. Alternatively, the joint can be locked by a manual action, e. g. by moving a bolt or the like. The joint may be releasable by an actuator, e. g. a solenoid or the like, or by manual action. This manual action can be a load to the joint, e.g. a moment of flexion or extension exceeding a defined value or a manual action, acting on a locking element, e.g. by moving a bolt or the like. After extending the prosthesis to its maximum position, the prosthesis may be hyper-extended to release the form fit locking and to bend the prosthesis to a more flexed position, vice versa. Preferably, the prosthesis in its maximum extended position will be flexed by exceeding a defined value of extension moment.

The joint can consecutively lock the external modular frame in a rest position and at least one functional position by means of a dorsal or palmar pressure of the distal part of the joint which will reach a release position in the event of hyper-flexion or hyper-bending, wherein the prosthesis is released to return to the rest position by means of elastic components, e.g. a spring or structural elements of the cover.

The joint or the bendable interface may contain a fail safety mechanism, too, wherein the fail safety mechanism allows - in various directions - hyper-extension or hyper-flexion, ductile deformation or is designed as a predetermined breaking point.

The fail safety mechanism may be a friction-type locking, e. g. a magnetic joint, or adhesive spring loaded form fit locking, a spring loaded hinge or a non-linear spring element.

The bendable interface may be an elastic element, e. g. a metal wire, which is encircled by tube-typed device to allow internal sliding of the plastic-elastic element upon bending and adapting the location of bending joint by adjustment of the length of the tube type device.

The tube-type device may be a spiral, which at its turn may be encircled by cylindrical components ensuring the location of possible flexion or extension.

The external modular frame or the coupling component may be produced from metal or plastic promoting, limiting or eliminating the conduction of vibrations to the intracorporeal bone structure. The external modular frame or the coupling component can be designed as a dampening device for vibrations. Specific components of the external modular frame or the coupling component may also limit the conduction of heat or cold to the intracorporeal implant and bone structure by thermal insulation, e.g. plastic elements.

The distal part of the external modular frame may have an extra-low hardness to promote larger surface-contact when getting in contact with objects. The functional or cosmetic cover may be loaded with agents leaving the cover and thus influencing the soft tissue surrounding the transcutaneous component.

A fail safety mechanism for a finger or toe prosthesis may have an external modular frame which is coupled to the intracorporeal implant by a friction type locking, e.g. a magnetic joint, a form fit locking, a spring loaded hinge or a non-linear-spring allowing hyper-extension or hyper-flexion, ductile deformation or breaking at a predetermined load.

Due to the modularity of the frame various functions, e.g. joints, coupling or fail-safety-components can be implemented and combined in an efficient and cost effective way, combined with the cover. Due to the joints or bending interfaces it is possible to adapt the form of the prosthesis to actual requirements. Furthermore, a fail-safety-mechanism allows the external modular frame to move relatively to the intracorporeal implant or to detach from the intracorporeal implant upon an overload so that the stress transferred by the external modular frame to the intracorporeal implant can be limited.

The invention is illustrated by the accompanying drawings in which:
- Figure 1: shows a schematic view of a finger prosthesis;
- Figure 2: shows a joint;
- Figure 3: shows a part of a joint according to figure 2;
- Figure 4: shows a first embodiment of a prosthesis.
- Figure 5: shows an alternative embodiment of the prosthesis; ,
- Figure 6: shows a prosthesis with a bendable interface;
- Figure 7: shows an example of a possible component configuration;
- Figure 8: shows a sectional view of a stump with an trancutaneous component and a cover or protective cap;
- Figure 9: shows an embodiment of an element of an aesthetic cover, modular frame or protective cap interacting with the skin and soft tissue of the stump; and
- Figure 10: shows an example for a prosthesis in situ.

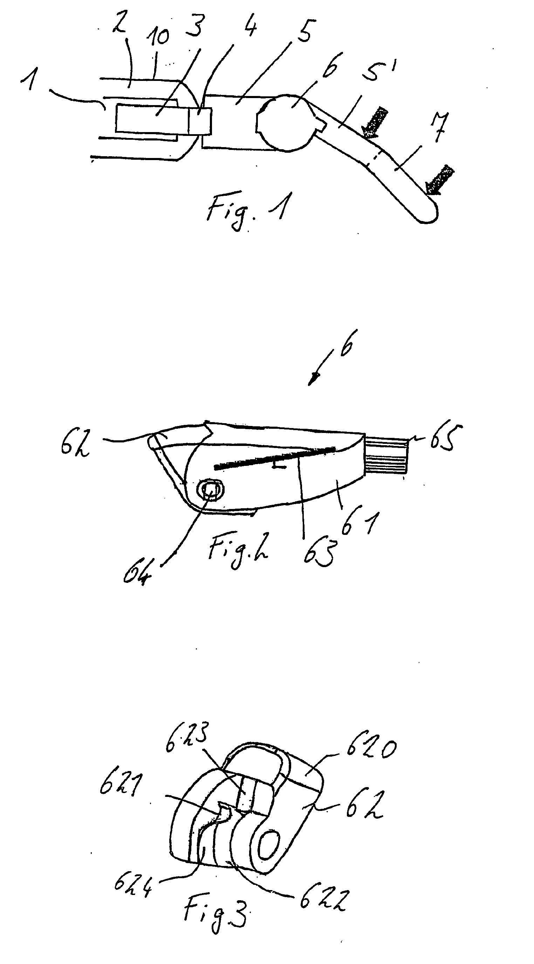

Referring to the drawings, figure 1 shows schematically a bone structure 1 surrounded by soft tissue 2 covered by a skin 10. Into the bone structure 1 an implant 3 is introduced in longitudinal manner. The implant 3 may be screwed into the remaining bone structure 1. Osseous integration will ensure an anchoring of the implant 1. A transcutaneous component 4, defining an abutment, perforates the skin 10 and offers the possibility of connecting other structures to the implant 3 and the stump. The transcutaneous component or abutment 4 may be an integral part of the implant 3 or is removably fixed to the implant 3. This implant 3 and the proximal intracorporeal part of the transcutaneous component 4 constitute the intracorporal level.

Onto the transcutaneous component 4 an extension as a coupling component 5 is removably fixed. The coupling component 5 is connected to the transcutaneous component 4. The coupling component 5 is the connection between the intracorporal level or intracorporal part and an extracorporal level of the trancutaneous component 4 and an external modular frame consisting of the coupling component 5, and joint 6 fixed to the coupling component 5 and extensions 5', 7, distally arranged to the joint 6.

The connection between the transcutaneous component 4 and the external modular frame, consisting of the coupling component 5, the joint 6 and the extensions 5', 7 needs to be sufficiently stiff to support the finger in movement. On the other hand, the patient needs to be able to remove and connect the external modular frame 5, 6, 5', 7 as a finger epithesis in a relatively simple manner, whether or not by means of using an accessory. The design of the coupling component 5 or interface may also include a fail safety mechanism function. Thus, in the event of an overload or hyper-rotation, the external modular frame 5, 6, 5', 7 and the intracorporal parts 3, 4 will be decoupled as to safeguard the bone structure 1 of trauma. Should the coupling component 5 not constitute part of the abutment 4 it may easily be removed by the patient, whether or not by means of using an accessory. The patient may also, if desired, cover the transcutaneous component 4 avoiding visibility of metal elements and to protect the transcutaneous component when the finger epithesis is being removed. In addition the coupling component 5 may also serve as an extension thus the level of the amputation may by mitigated whereby the joint 6 will be placed at an ideal position. Consequently, the coupling component 5 will be produced in various lengths.

The external modular frame 5, 6, 5', 7 constitutes the extension of the bone structure 1 of the amputated finger. The frame is preferably composed of a light and strong material, e. g. alloys such as titanium grade 5 or any other suitable material. The material may be selected in accordance to the preferences of the patient. If the patient feels comfortable to receive vibrations from the external modular frame to the intracorporal implant 3 and bone structure 1, the material is selected to transmit such vibrations. Vibrations from the external frame may promote osseous perception. If a patient does not prefer to receive vibrations from the external frame, the coupling component 5 or the selected materials may limit or eliminate such vibrations. The selection of material determines the conduction of heat or cold as well. For this reason, insulation materials, e.g. ceramics or plastics can be used in combination with their properties of vibration conduction.

Figure 2 shows an example of the joint 6 consisting of a first articulation 61 and a second articulation 62. A spring 63 is connected into the second articulation 62 and runs over the first articulation 61. The position of the spring 63 is crucial as to the functioning of the joint 6 or hinge. The articulations 61, 62 are interconnected by means of an axis 64 which constitutes the rotation axis of the joint 6.

A fastening device 65 is attached to the first articulation 61 to receive an extension 5' or the like. A not shown coupling component 5 may be connected to the second articulation 62.

The joint 6 is a modular joint which, on the proximal side, contains a matrix of the not shown coupling component and whereto, on the distal side, the extension 5' may be connected. A different version may have the same design with the exception of the possibility of connecting a distal extension and the saving in respect of an artificial fingernail.

The extension 5' extends to the joint 6 distally and consists of a structure of a free from shape which is set at a whether or not determined angle and of which the lengths may be vary. At the end of the distal part room will be created for a nail. An antl-rotation system will avoid movement, especially rotation around the longitudinal axis. The whole will be anchored to the joint 6.

Particular interest is paid to the movement of the joint 6. By fixing the external modular frame 5, 6, 5', 7 into various different positions, the finger prosthesis becomes functional. The selection of the material of the external modular frame 5, 6, 5', 7 and the fixation of the frame onto the intracorporal components 3, 4 by means of the coupling component 5 will ensure transmission of forces and, if desired, vibrations caught by the surface of the prosthesis material and translated to the bone structure 1 by means of the frame.

The joint 6 may have a rest position, functional position and a resetting position. Exercising downward pressure on the dorsal side, as indicated by the arrows in figure 1, distally of the joint axis 64 will cause a transition from the rest position to the functional position (flexion). This position will automatically be locked thus the finger will be set to an active functional position. To leave the functional position again, a downward pressure will be exercised distally of the joint axis which will set the extensions 5', 7 to a hyper-flexion and will unlock and reset the system. An aesthetic cover consisting of elastic material such as foam or soft silicone or elastic components of the modular frame will function as a spring and will automatically set the frame from the unlocking position to the rest position. The joint 6 may also contain a fail safety mechanism which will dissipate the energy caused by an overload or hyper-rotation. In this case, the spring 63 buckles due to overload or a control slipping of cylindrical components of the modular frame, joint and cosmetic cover takes place.

Figure 3 shows the second first articulation 62 with an outer frame 620 showing an anchoring position or functional position 621, an unlocking course 624 in case of hyper-flexion, a return course to the rest position 622 and a rest position spring 623.

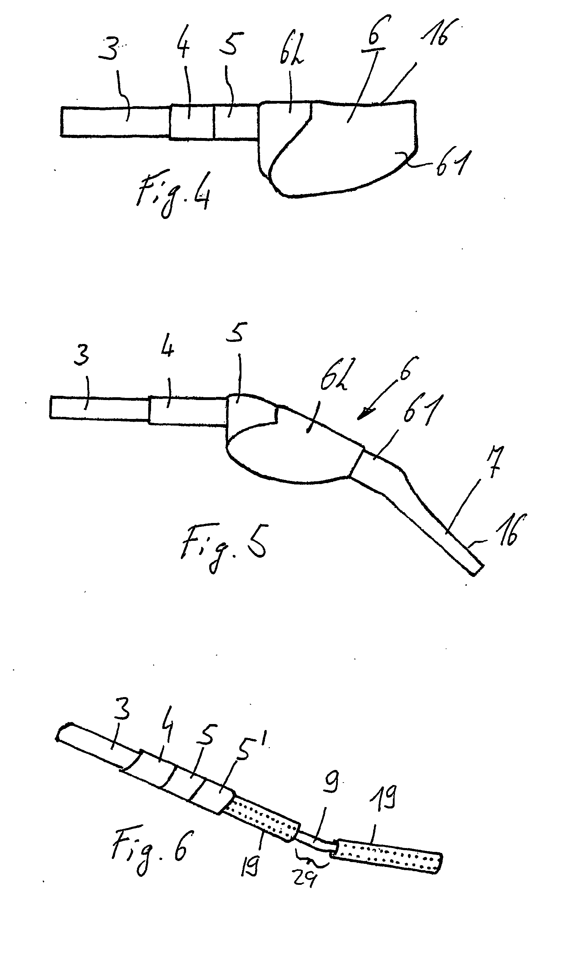

In Figure 4 a finger prosthesis s shown for replacement of a bone structure in the event of an amputation at the level of the medial phalanx or in the event of an amputation through the proximal-inter-phalangeal joint (PIP). The implant 3 and the transcutaneous component are connected to the coupling component 5 and the joint 6, wherein the distal part 61 of the joint 6 has on its upper surface a saving 16 for a nail prosthesis.

Figure 5 shows an alternative embodiment with an implant 3 and a transcutaneous component 4. The coupling component 5 is part of the joint 6.

The joint 6 is extended by the extension 7 to distal. The external modular frame 5, 6, 7 can be used after amputation at a level of the proximal phalanx, in the event of amputation of the medial or distal phalanx or in the event of amputation through the proximal or distal inter phalanx joint.

Figure 6 shows an alternative embodiment with the implant 3 and transcutaneous component 4, connected to the coupling component 5, which acts at the same time as an extension. The coupling component 5 is designed as a male joint component, wherein a corresponding female component acts as an extension 5'. To bend a distal part of the finger prosthesis a metal wire 9 is connected to the coupling component 5 via extension 5'. Folding or bending the metal wire 9 will allow the finger prosthesis to change shape. Around the metal wire 9 a tubular device 19 is arranged. Alternatively, a spring or spiral may encircle the metal wire 9. A fissure 29 is defined between the tubular elements 19. The fissure 29 reduces the bending stiffness allowing bending and allows further the metal wire 9 to move freely within the prosthesis at flexion or extension. Cylindrical structures may be positioned over the metal wire 9 and anchored thus the metal wire 9 will only bend when free. In this manner the exact location of the bending joint may be determined. The position of the prosthesis may be changed manually. The resistance or bending stiffness of the metal wire 9, constituting as a core of the external frame, needs to be sufficient high to allow transfer of loads but need to be sufficient flexible to ensure the function of a bending joint. Alternatively, the bending joint is used for adaption of the flexion angle of the metal wire 9 only. The bending of the metal wire 9 additionally functions as a fail safety mechanism, the bending capacities will dissipate the energy caused by an overload or hyper-rotation. The metal wire 9 may be elastic-plastic to allow permanent bending flexion.

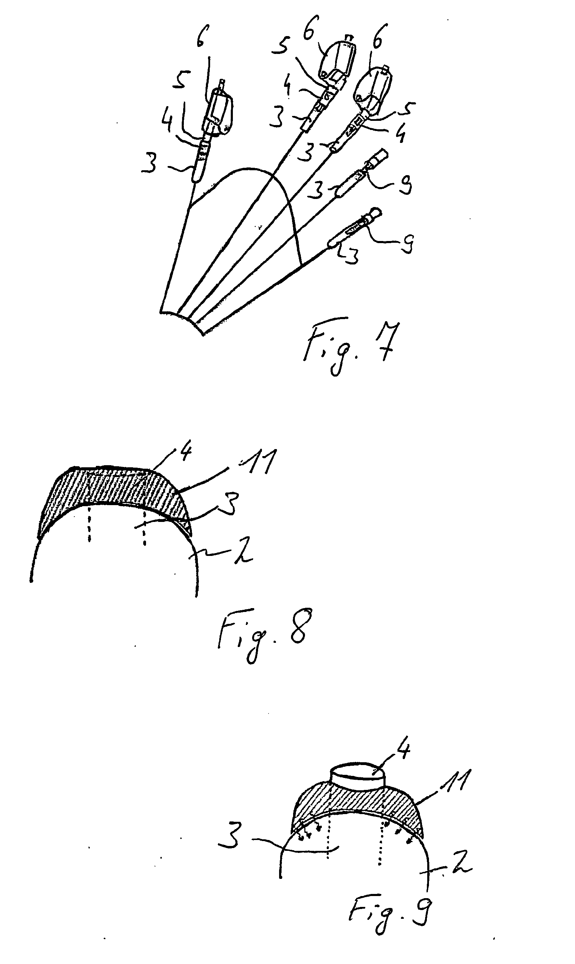

In Figure 7 an example of a possible component configuration is shown wherein for a thumb a prosthesis according to figure 4 is used preferably, A prosthesis according to figure 5 may be used for the index finger and the middle finger, because these three fingers are more often used than the ring finger or little finger. Because of the frequent use of the first three fingers, a joint 6 with a hinge and a locking mechanism as well as a fail safety mechanism is more stable and reliable. The ring finger and the little finger may be equipped with a prosthesis according to figure 6, in which a fail safety mechanism is realized by elastic-plastic deformation of the metal wire 9 or an other elastic-plastic component.

Figure 8 shows a sectional view through an amputated finger with an implant 3 and a transcutaneous component encircled by soft tissue 2 of the stump. The external modular frame 5, 6, 5', 7 is removed from the transcutaneous component and for protection and/or for aesthetic reasons a protective cap or cosmetic cover 11 is arranged onto the transcutaneous component 4. The cover 11 may contain an agent or agents leaving the cover 11 and thus influencing the soft tissue 2 surrounding the transcutaneous component 4 to stimulate the soft tissue 2 or the skin 10. An alternative embodiment is shown in Figure 9 in which the cover 11 encircles the transcutaneous component 4 which passes through the cover 11. The cover 11 exercises pressure on the soft tissue 2 surrounding the transcutaneous component 4 as indicated by the arrows to influence the soft tissue and skin, e.g. healing, growth and proprioception. The cover can be a part of the extracorporeal modular frame and its cover as well.

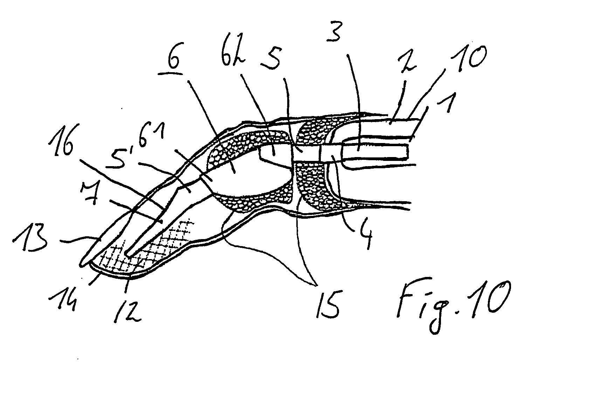

Figure 10 shows in a cross sectional a view of a prosthesis with the Intracorporal implant 3 which is integrated in a remaining bone structure 1 of a finger. The transcutaneous component 4, surrounded by the soft tissue 2 is coupled via the coupling component 5 which is in turn coupled to the proximal part 62 of the joint 6. The distal part 61 of the joint 6 is connected to an extension 5' and a second distal extension 7 with a saving 16 for a fingernail prosthesis 13. Around the external modular frame 5, 6, 5', 7 a soft material 12 is arranged, enhancing the grip. The soft material 12 has an extra-low hardness to promote a larger surface contact when gripping or getting in contact with objects. The extended modular frame 5, 6, 5', 7 is surrounded partially by foam 15 or other elastic materials to achieve a natural appearance of an outer artificial skin in respect to wrinkling, elongation, shortening or buckling of the skin 14 of the finger prosthesis. These elastic materials 15 may have various physical characteristics and may promote a return movement into a rest position after reaching a release position upon bending of joint 6.

The modular frame 5, 6, 5', 7 largely determines the level of functionality of the finger prosthesis. The cosmetics of the finger prosthesis on the other hand will be determined by the aesthetic or cosmetic finishing of the elastic material 15 and the artificial skin 14 as well as the soft material 12. The aesthetic and functional cover 12, 15, 14 may be made of materials that shall remain smooth such as polyurethane or silicone elastomers. The nail prosthesis 13 may be created from a smooth or hard material and may be connected to the aesthetic cover in mechanical and/or chemical manner. The characteristics of the selected materials form the aesthetic and functional cover depend on the position within the finger. Near to the joint 6 a material with a high level of elasticity and a low E-modulus may be used. Near the top of the finger (distal end) a smooth material 12 with a low hardness and with high resistance against abrasion may be selected. The proximal part of the prosthesis may be made of a material with a high resistance against rubbing and tearing. The whole may be covered with a thin colored or transparent layer 14 with a high level of abrasion resistance. Near the joint 6 the aesthetic cover will be connected to the frame by the foam material 15 which causes the elasticity of the prosthesis material at a flexion to be limited to the region between both fixations 61, 62. The room between the cosmetic cover and the modular frame 5, 6, 5', 7 may eventually be filled by means of a soft form material or any other soft filling material. The low level of hardness of the prosthesis material shall also have a function. Positioning itself around the object to be taken point contact will be replaced by surface contact which will tighten the grip. However, the modular frame may also be used without the aesthetic covers 12, 13, 14 solely covered by means of a protective cover thus exclusively using the functional characteristics of the system.

## Claims

1. A finger or toe prosthesis having an intracorporeal implant (3) and an external modular frame (5, 6, 7, 5') constituting the core of the finger or toe prosthesis with a number of modules or components, each with its own function, wherein the external modular frame (5, 6, 7, 5') is displaceably fixed on a trancutaneous component (4) of the intracorporeal implant (3) to form one modular frame (5, 6, 7, 5'), the external modular frame comprising a coupling component (5) for coupling the intracorporeal implant (3) to the external modular frame, **characterized by** a joint (6) or bendable interface (9), and a distal extension (5') connected to the joint (6) or bendable interface (9), wherein the external modular frame (5, 6, 7, 5') is covered with a cosmetic or functional cover (12, 13, 14, 15) consisting of a smooth material fixed to the external modular frame (5, 6, 7, 5').

2. A finger or toe prosthesis according to claim 1, wherein the coupling component (5) is an elastic component.

3. A finger or toe prosthesis according to claim 1 or 2, wherein the coupling component (5) is an adhesive or a snap joint.

4. A finger or toe prosthesis according to one of the preceding claims, wherein the coupling component (5) contains an overload protection device.

5. A finger or toe prosthesis according to one of the preceding claims, wherein the coupling component (5) is removably attached to the implant (3), covering the transcutaneous component (4).

6. A finger or toe prosthesis according to one of the preceding claims, wherein the material of the coupling component which is capable of getting in contact with the skin (10) or soft tissue (2) of the patient is loaded with an agent capable of leaving the material and influencing the skin (10) or soft tissue (2) surrounding the transcutaneous component (4).

7. A finger or toe prosthesis according to one of the preceding claims, wherein the joint (6) is lockable in a position by a form fit locking upon reaching a predetermined angle position or a manual action.

8. A finger or toe prosthesis according to claim 7, wherein the joint (6) is releasable by an actuator.

9. A finger or toe prosthesis according to one of the preceding claims, wherein the joint (6) consecutively locks the external modular frame (5, 6, 7, 5') in a rest position and in one or more functional positions by means of a dorsal or palmar pressure of the distal part (5', 7) of the joint (6), and is capable of reaching a release position in the event of hyper-flexion or hyper-bending, wherein it is released to return to the rest position by means of a spring (63).

10. A finger or toe prosthesis according to one of the preceding claims, wherein the joint (6) or bendable interface (9) contains an overload protection device.

11. A finger or toe prosthesis according to claim 4 or 10, wherein the overload protection device allows hyper-extension or hyper-flexion or is designed as a predetermined breaking point.

12. A finger or toe prosthesis according to claim 4, 10 or 11, wherein the overload protection device is a friction locking, e.g. a magnetic joint, a spring loaded form fit locking, a spring loaded hinge or a non-linear-spring.

13. A finger or toe prosthesis according to one of the preceding claims, wherein the bendable interface (9) is an elastic-plastic element, e.g. a metal wire, which is encircled by a tubular device (19) to allow internal sliding of the elastic-plastic element upon bending and adapting the location (29) of the bending joint by adjustment of the length of the tubular device (19).

14. A finger or toe prosthesis according claim 13, wherein the tubular device (19) is a spiral, which at its turn may be encircled by cylindrical components ensuring the location (29) of possible flexion.

15. A finger or toe prosthesis according to claim 1, wherein the external modular frame (5, 6, 7, 5') is completely covered by the cover (12, 13, 14, 15).

16. A finger or toe prosthesis according to claim 15, wherein the cover (12, 13, 14, 15) of the modular frame (5, 6, 7, 5') is locally composed of various elements, specifically attached with each other with various physical characteristics to achieve a natural appearance in respect to wrinkling, elongation, shortening or buckling.

17. A finger or toe prosthesis according to claim 16, wherein the cover (12, 13, 14, 15) is made of foam or soft silicone for supporting the underlying external modular frame (5, 6, 7, 5').

18. A finger or toe prosthesis according to one of the preceding claims, wherein a distal part of the external modular frame (5, 6, 7, 5') has a lower hardness for promoting a larger surface-contact when gripping or getting in contact with objects.

## Patentansprüche

1. Eine Finger- oder Zehenprothese mit einem intrakorporalen Implantat (3) und einem externen modularen Rahmen (5, 6, 7, 5') der den Kern der Finger- oder Zehenprothese ausbildet, mit einer Anzahl von Modulen oder Komponenten, jedes oder jede mit deren eigener Funktion, wobei der externe modulare Rahmen (5, 6, 7 5') entfernbar an einer transkutanen Komponente (4) des intrakorporalen Implantats (3) befestigt ist, um einen modularen Rahmen auszubilden, der externe modulare Rahmen weist eine Kopplungskomponente (5) zum Koppeln des intrakorporalen Implantats (3) an den externen modularen Rahmen auf, **gekennzeichnet durch** ein Gelenk (6) oder biegbares Interface (9) und eine distale Verlängerung (5'), die mit dem Gelenk (6) oder dem biegbaren Interface (9) verbunden ist, wobei der externe modulare Rahmen (5, 6, 7 5') mit einer kosmetischen oder funktionalen Hülle (12, 13, 14, 15) abgedeckt ist, die aus einem weichen Material besteht, das an dem externen modularen Rahmen (5, 6, 7, 5') befestigt ist.

2. Eine Finger- oder Zehenprothese nach Anspruch 1, wobei die Kopplungskomponente (5) eine elastische Komponente ist.

3. Eine Finger- oder Zehenprothese nach Anspruch 1 oder 2, wobei die Kopplungskomponente (5) ein Klebstoff oder ein Schnappgelenk ist.

4. Eine Finger- oder Zehenprothese nach einem der voranstehenden Ansprüche, wobei die Kopplungskomponente (5) eine Überlastschutzvorrichtung enthält.

5. Eine Finger- oder Zehenprothese nach einem der voranstehenden Ansprüche, wobei die Kopplungskomponente (5) entfernbar an dem Implantat (3), die transkutane Komponente (4) abdeckend, angeordnet ist.

6. Eine Finger oder Zehenprothese nach einem der voranstehenden Ansprüche, wobei das Material der Kopplungskomponente, das dazu in der Lage ist, mit der Haut (10) oder dem Weichgewebe (2) des Patienten in Kontakt zu treten, mit einem Wirkstoff beschickt ist, der in der Lage ist, das Material zu verlassen und die Haut (10) oder das Weichgewebe (2), die die transkutane Komponente (4) umgeben, zu beeinflussen.

7. Eine Finger- oder Zehenprothese nach einem der voranstehenden Ansprüche, wobei das Gelenk (6) in einer Stellung durch eine formschlüssige Verriegelung nach Erreichen einer vorbestimmten Winkelstellung oder eine manuelle Einwirkung verriegelbar ist.

8. Eine Finger- oder Zehenprothese nach Anspruch 7, wobei das Gelenk (6) durch einen Aktuator lösbar ist.

9. Eine Finger- oder Zehenprothese nach einem der voranstehenden Ansprüche, wobei das Gelenk (6) nacheinander den externen modularen Rahmen (5, 6, 7, 5') in einer Ruhestellung und in einer oder mehreren funktionalen Stellungen mittels eines dorsalen oder palmaren Druckes auf den distalen Teil (5', 7) des Gelenkes (6) verriegelt und in der Lage ist, eine Lösestellung im Falle einer Tdyper-Flexion oder einer Flyper-Beugung zu erreichen, wobei es gelöst wird, um mittels einer Feder (63) in die Ruhestellung zurückzukehren.

10. Eine Finger- oder Zehenprothese nach einem der voranstehenden Ansprüche, wobei das Gelenk (6) oder biegbare Interface (9) eine Überlastschutzvorrichtung aufweist.

11. Eine Finger- oder Zehenprothese nach Anspruch 4 oder 10, wobei die Überlastschutzvorrichtung eine Hyperextension oder Hyperflexion ermöglicht oder als eine Sollbruchstelle ausgebildet ist.

12. Eine Finger- oder Zehenprothese nach Anspruch 10 oder 11, wobei die Überlastschutzvorrichtung eine Reibschlussverbindung ist, zum Beispiel ein Magnetgelenk, eine federvorgespannte Formschlussvewiegelung, ein federvorgespanntes Gelenk oder eine nicht lineare Feder.

13. Die Finger- oder Zehenprothese nach einem der voranstehenden Ansprüche, wobei das biegbare Interface (9) ein elastisch-plastisches Element ist, zum Beispiel ein Metalldraht, der von einer röhrenförmigen Vorrichtung (19) umgeben ist, um ein internes Gleiten des elastisch-plastischen Elementes beim Biegen und Anpassen der Stelle (29) des sich biegenden Gelenkes durch Einstellung der Länge der rohrförmigen Vorrichtung (19) zu erlauben.

14. Eine Finger- oder Zehenprothese nach Anspruch 13, wobei die rohrförmige Vorrichtung (19) eine Spirale ist, die ihrerseits durch zylindrische Komponenten umgeben sein kann, die die Stelle (29) einer möglichen Flexion sicherstellt.

15. Eine Finger- oder Zehenprothese nach Anspruch 1, wobei der externe modulare Rahmen (5, 6, 7, 5') vollständig durch die Hülle (12, 13, 14, 15) abgedeckt ist.

16. Eine Finger- oder Zehenprothese nach Anspruch 15, wobei die Hülle (12, 13, 14, 15) des modularen Rahmens (5, 6, 7, 5') lokal aus verschiedenen Elementen zusammengesetzt ist, speziell aneinander befestigt, mit verschiedenen physikalischen Eigenschaften, um eine natürliche Erscheinung in Einsicht auf Faltensclalagen, Verlängerung, Verkürzung oder Ausbeulen zu erreichen.

17. Eine Finger- oder Zehenprothese nach Anspruch 16, wobei die Hülle (12, 13, 14, 15) aus einem Schaum oder einem weichen Silikon zum Abstützen des unterliegenden externen modularen Rahmens 5, 6, 7, 5' hergestellt ist.

18. Eine Finger- oder Zehenprothese nach einem der voranstehenden Ansprüche, wobei ein distales Teil des externen modularen Rahmens (5, 6, 7, 5') eine geringere Härte zum Bereitstellen eines größeren Oberflächenkontaktes während des Greifens oder beim in Kontakt treten mit Objekten aufweist.

## Revendications

1. Prothèse de doigt ou d'orteil comprenant un implant (3) intracorporel et un châssis modulaire externe (5, 6, 7, 5') constituant le coeur de la prothèse de doigt ou d'orteil, ayant un certain nombre de modules ou composants, chacun ayant sa propre fonction, le châssis modulaire externe (5, 6, 7, 5') étant fixé de façon déplaçable sur un composant transcutané (4) de l'implant intracorporel (3) pour former un châssis modulaire (5, 6, 7, 5'), le châssis modulaire externe comprenant un composant de couplage (5) pour coupler l'implant intracorporel (3) au châssis modulaire externe, **caractérisé par** un joint (6) ou interface capable de cambrage (9), et une extension distale (5') reliée au joint (6) ou interface capable de cambrage (9), le châssis modulaire externe (5, 6, 7, 5') étant recouvert par un revêtement (12, 13, 14, 15) cosmétique ou fonctionnel consistant en un matériau doux fixé au châssis modulaire externe (5, 6, 7, 5').

2. Prothèse de doigt ou d'orteil selon la revendication 1, dans laquelle le composant de couplage (5) est un composant élastique.

3. Prothèse de doigt ou d'orteil selon la revendication 1 ou 2, dans laquelle le composant de couplage (5) est un adhésif ou un joint à encliqueter.

4. Prothèse de doigt ou d'orteil selon l'une des revendications précédentes, dans laquelle le composant de couplage (5) contient un dispositif de protection contre les surcharges.

5. Prothèse de doigt ou d'orteil selon l'une des revendications précédentes, dans laquelle le composant de couplage (5) est attaché de façon amovible à l'implant (3), recouvrant le composant transcutané (4).

6. Prothèse de doigt ou d'orteil selon l'une des revendications précédentes, dans laquelle le matériau du composant de couplage, lequel est capable d'entrer en contact avec la peau (10) ou un tissu mou (2) du patient, est chargé avec un agent capable de quitter le matériau et d'influencer la peau (10) ou le tissu mou (2) entourant le composant transcutané (4).

7. Prothèse de doigt ou d'orteil selon l'une des revendications précédentes, dans laquelle le joint (6) est verrouillable dans une position par un verrouillage par formes conjuguées une fois atteinte une position angulaire prédéterminée, ou par une action manuelle.

8. Prothèse de doigt ou d'orteil selon la revendication 7, dans laquelle le joint (6) est libérable par un actionneur.

9. Prothèse de doigt ou d'orteil selon l'une des revendications précédentes, dans laquelle le joint (6) verrouille consécutivement le châssis modulaire externe (5, 6, 7, 5') dans une position de repos et dans une ou plusieurs positions fonctionnelles, au moyen d'une pression dorsale ou palmaire de la partie distale (5', 7) du joint (6), et est capable d'atteindre une position de libération dans le cas d'une hyper-flexion ou hyper-cambrage, dans laquelle celle-ci est libérée pour retourner à la position de repos au moyen d'un ressort (63).

10. Prothèse de doigt ou d'orteil selon l'une des revendications précédentes, dans laquelle le joint (9) ou l'interface capable de cambrage (9) contient un dispositif de protection contre les surcharges.

11. Prothèse de doigt ou d'orteil selon la revendication 4 ou 10, dans laquelle le dispositif de protection contre les surcharges permet une hyper-extension ou une hyper-flexion ou est conçu en tant que point de rupture prédéterminé.

12. Prothèse de doigt ou d'orteil selon la revendication 10 ou 11, dans laquelle le dispositif de protection contre les surcharges est un verrouillage par friction, par exemple un joint magnétique, un verrouillage par formes conjuguées chargé par un ressort, une charnière chargée par un ressort ou un ressort non linéaire.

13. Prothèse de doigt ou d'orteil selon l'une des revendications précédentes, dans laquelle l'interface capable de cambrage (9) est un élément plastique-élastique, par exemple un fil métallique, lequel est encerclé par un dispositif tubulaire (19) pour permettre un coulissement interne de l'élément plastique-élastique lors du cambrage, et l'adaptation de l'emplacement (29) du joint capable de cambrage par ajustement de la longueur de dispositif tubulaire (19).

14. Prothèse de doigt ou d'orteil selon la revendication 13, dans laquelle le dispositif tubulaire (19) est une spirale, laquelle peut être à son tour encerclée par des composants cylindriques assurant l'emplacement (29) de la flexion possible.

15. Prothèse de doigt ou d'orteil selon la revendication 1, dans laquelle le châssis modulaire externe (5, 6, 7, 5') est entièrement recouvert par le revêtement (12, 13, 14, 15).

16. Prothèse de doigt ou d'orteil selon la revendication 15, dans laquelle le revêtement (12, 13, 14, 15) du châssis modulaire (5, 6, 7, 5') est localement composé de divers éléments attachés spécifiquement les uns avec les autres avec diverses caractéristiques physiques, pour réaliser une apparence naturelle en termes de plissement, d'élongation, de raccourcissement ou de gonflement.

17. Prothèse de doigt ou d'orteil selon la revendication 16, dans laquelle le revêtement (12, 13, 14, 15) est en mousse ou en silicone mou pour supporter le châssis modulaire externe (5, 6, 7, 5') sous-jacent.

18. Prothèse de doigt ou d'orteil selon l'une des revendications précédentes, dans laquelle la partie distale du châssis modulaire externe (5, 6, 7, 5') a une dureté réduite pour favoriser un plus grand contact surfacique lors de la préhension ou de l'entrée en contact avec des objets.
